# EUROPEAN PATENT APPLICATION

(11) **EP 4 796 162 A1**
(43) Date of publication of application: **26.08.2026**
(21) Application number: 24879916.5
(22) Date of filing: 12.08.2024
(51) Int. Cl.: A61N 1/32, A61N 1/40, A61N 1/36, A61N 1/04

(54) **SKINCARE DEVICE**

(30) Priority: 20.10.2023 KR 20230141314; 16.01.2024 KR 20240006883
(71) Applicant: APR CO., LTD., Seoul 05551 (KR)
(72) Inventor: LEE, Gyoun Jung, Seoul 05551 (KR); PARK, Seung Woo, Seoul 05551 (KR)
(74) Representative: SONN Patentanwälte GmbH & Co KG
(86) International application number: PCT/KR2024/011981
(87) International publication number: WO 2025/084576

(57) **Abstract**

A skincare device is disclosed. The skincare device according to an aspect of the present invention may comprise: a first electrode and a second electrode that are brought into contact with the skin; and an electrode voltage application unit that selectively implements one of a first mode in which a first voltage is applied between the first electrode and the second electrode and a second mode in which a second voltage greater than the first voltage is applied to at least one of the first electrode and the second electrode.

## Description

### TECHNICAL FIELD

The present invention relates to a skincare device.

### BACKGROUND

A skincare device using electrical stimulation may have different effects on skincare depending on a configuration of electrodes and a type of stimulation intensity.

Cosmetic devices having electrodes of unipolar, bipolar, or monopolar types have been manufactured and sold on the market.

Effects applied to the skin vary depending on a type, voltage, and frequency of electrical stimulation, and since facial skin is thin and highly sensitive, electrical stimulation having an appropriate voltage and frequency should be properly applied in order to obtain a desired skincare effect.

A bipolar-type cosmetic device is used in a manner in which a low voltage is applied between two electrodes and the two electrodes to which the low voltage is applied are brought into contact with the skin.

The bipolar-type cosmetic device provides so-called MC (Microcurrent) electrical stimulation, EMS (Electrical Muscle Stimulation) electrical stimulation, and EP (Electroporation) electrical stimulation to the skin, and in this case, skin health is improved by using low-voltage electrical stimulation on the order of several tens of volts.

A unipolar-type cosmetic device is used in a manner in which a single electrode to which a high voltage is applied is brought into contact with the skin. In this case, the high voltage applied to the single electrode refers to a voltage between a ground portion and the single electrode.

The unipolar-type cosmetic device applies so-called RM (Radiofrequency Microporation) electrical stimulation to the skin, and in this case, minimally invasive skin stimulation is induced using a high voltage corresponding to several thousand volts and a high-frequency stimulation on the order of tens of thousands to hundreds of thousands of hertz, which induces enhanced delivery of effective water-soluble drugs and also includes a recovery process of skin damage caused by invasive stimulation.

In the case of a cosmetic device including a function of RM (Radiofrequency Microporation) electrical stimulation that causes microporation beyond an action mechanism for inducing absorption of an active ingredient, an optimal skincare effect may be obtained only when a high voltage is used and is fully delivered to the skin at a high frequency.

Conventional bipolar-type and unipolar-type skincare devices have different operating voltages, and thus are manufactured as separate devices, or even when manufactured as a single device, separate transformers for high voltage and low voltage are required, resulting in an increase in size of the device.

In particular, since the unipolar-type skincare device uses a high voltage, there is a problem in that a size of a skin contact detection unit increases in consideration of stability and reliability of the device.

### DISCLOSURE

### TECHNICAL PROBLEM

An object of the present invention is to provide a skincare device capable of selectively providing a bipolar-type operation and a unipolar-type operation.

Another object of the present invention is to provide a skincare device configured such that a detection unit 410 for detecting whether the skin is in contact during the bipolar-type operation is used during the unipolar-type operation.

### TECHNICAL SOLUTION

According to an aspect of the present invention, provided is a skincare device, including: a first electrode and a second electrode configured to contact skin; and an electrode voltage application unit configured to selectively implement one of a first mode in which a first voltage is applied between the first electrode and the second electrode and a second mode in which a second voltage greater than the first voltage is applied to at least one of the first electrode and the second electrode.

The electrode voltage application unit may include a transformer including a primary coil to which an input voltage is applied, a secondary coil configured to output the first voltage induced by the primary coil, and a tertiary coil configured to output the second voltage induced by the primary coil, one output terminal of the tertiary coil being grounded; a relay including a first movable contact and a second movable contact respectively connected to two output terminals of the secondary coil, a third movable contact and a fourth movable contact respectively connected to a first branch output terminal and a second branch output terminal branched from another output terminal of the tertiary coil, and a first fixed contact and a second fixed contact respectively connected to the first electrode and the second electrode; and a first transistor having an emitter connected to a relay coil of the relay and a collector grounded, wherein, according to a control signal received at a base of the first transistor, the first movable contact is connected to the first fixed contact and the second movable contact is connected to the second fixed contact to implement the first mode, or the third movable contact is connected to the first fixed contact and the fourth movable contact is connected to the second fixed contact to implement the second mode.

The electrode voltage application unit may include: a transformer including a primary coil to which an input voltage is applied, a secondary coil configured to output the first voltage induced by the primary coil, and a tertiary coil configured to output the second voltage induced by the primary coil, one output terminal of the tertiary coil being grounded; a relay including a first movable contact and a second movable contact respectively connected to two output terminals of the secondary coil, a third movable contact connected to another output terminal of the tertiary coil, and a first fixed contact and a second fixed contact respectively connected to the first electrode and the second electrode; and a first transistor having an emitter connected to a relay coil of the relay and a collector grounded, wherein, according to a control signal received at a base of the first transistor, the first movable contact is connected to the first fixed contact and the second movable contact is connected to the second fixed contact to implement the first mode, or the third movable contact is connected to one of the first fixed contact and the second fixed contact to implement the second mode.

In this case, the first coil may include a first-1 coil and a first-2 coil connected in series, the first coil includes first to third input terminals, the first input terminal and the second input terminal may serve as input terminals of the first-1 coil, and the second input terminal and the second input terminal may serve as input terminals of the first-2 coil, and the input voltage may be applied between any two selected from the first to third input terminals.

Meanwhile, the skincare device may further include a mode input unit configured to receive, from a user, a selected mode selected from the first mode and the second mode; and a controller configured to control the electrode voltage application unit according to the selected mode.

Meanwhile, the skincare device may further include a detection unit configured to detect whether the first electrode and the second electrode are in contact with skin in the first mode; and a switching unit configured to switch the detection unit in the second mode such that the detection unit detects whether an electrode, to which the second voltage is applied, among the first electrode and the second electrode is in contact with the skin.

In this case, the detection unit may include a photocoupler, and a light emitting unit of the photocoupler may be connected in series with one of two output terminals of the secondary coil.

Meanwhile, the detection unit may be connected to one of two output terminals of the secondary coil.

Meanwhile, the switching unit may include a second transistor having a collector connected to the detection unit and an emitter grounded.

Meanwhile, the skincare device may further include a mode input unit configured to receive, from a user, a selected mode selected from the first mode and the second mode; and a controller configured to control the switching unit according to the selected mode.

### ADVANTAGEOUS EFFECT

According to the above configuration, the skincare device according to an aspect of the present invention includes an electrode voltage application unit configured to selectively implement one of a first mode in which a first voltage is applied between a first electrode and a second electrode and a second mode in which a second voltage greater than the first voltage is applied to at least one of the first electrode and the second electrode, and thus can selectively implement the first mode, for example, a bipolar mode, and the second mode, for example, a unipolar mode, thereby being effective and useful.

In particular, since the transformer of the electrode voltage application unit has a structure in which a first coil, a second coil, and a third coil share a core, the first voltage and the second voltage can be output by a single transformer, and thus a structure of the skincare device is simplified and miniaturization is enabled as compared with a case in which separate transformers are used to output the first voltage and the second voltage.

In addition, a detection unit used to detect whether skin contact is made in the first mode can also be used to detect whether skin contact is made in the second mode, thereby simplifying a structure of the skincare device and facilitating manufacture thereof.

Advantageous effects of the present invention are not limited to the above-described effects, and it should be understood that the present invention includes all effects that can be inferred from the configurations described in the detailed description or claims.

### BRIEF DESCRIPTION OF THE DRAWINGS

FIG. 1 is a view illustrating a skincare device according to an embodiment of the present invention.
FIG. 2 is a view for explaining a configuration of a skincare device according to an embodiment of the present invention.
FIG. 3 is a circuit diagram of an electrode voltage application unit, a detection unit, and a switching unit according to a first embodiment of the present invention.
FIG. 4 is a view illustrating an example of the detection unit shown in FIG. 3.
FIG. 5 is a circuit diagram illustrating an electrode voltage application unit according to a second embodiment of the present invention.

### MODES OF THE INVENTION

Hereinafter, embodiments of the present invention will be described in detail with reference to the accompanying drawings so that those of ordinary skill in the art can readily implement the present invention. The present invention may be embodied in many different forms and is not limited to the embodiments set forth herein. Portions irrelevant to the description have been omitted for clarity of explanation of the present invention, and throughout the specification, the same reference numerals are used to denote identical or similar components.

The words and terms used in the present specification and claims should not be interpreted as being limited to their ordinary or dictionary meanings, but should be construed as having meanings and concepts consistent with the technical spirit of the present invention, in accordance with the principle that an inventor may define terms and concepts to best describe their invention.

Accordingly, the embodiments described in the present specification and the configurations shown in the drawings correspond to preferred embodiments of the present invention, and do not represent all the technical spirit of the present invention, so the configurations may have various examples of equivalent and modification that can replace them at the time of filing the present invention.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

The presence of an element in/on "front", "rear", "upper or above or top" or "lower or below or bottom" of another element includes not only being disposed in/on "front", "rear", "upper or above or top" or "lower or below or bottom" directly in contact with other elements, but also cases in which another element being disposed in the middle, unless otherwise specified. In addition, unless otherwise specified, that an element is "connected" to another element includes not only direct connection to each other but also indirect connection to each other.

Hereinafter, a skincare device 10 of the present invention will be described with reference to the accompanying drawings.

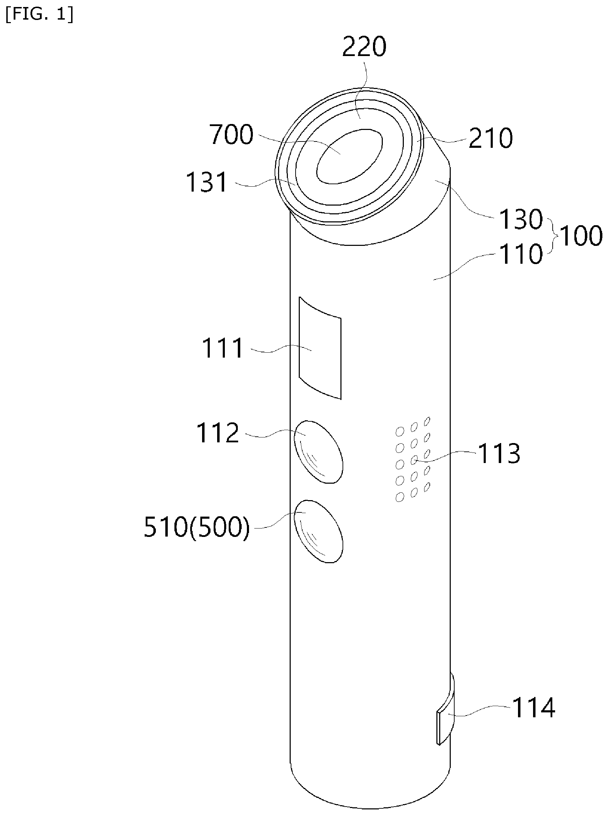

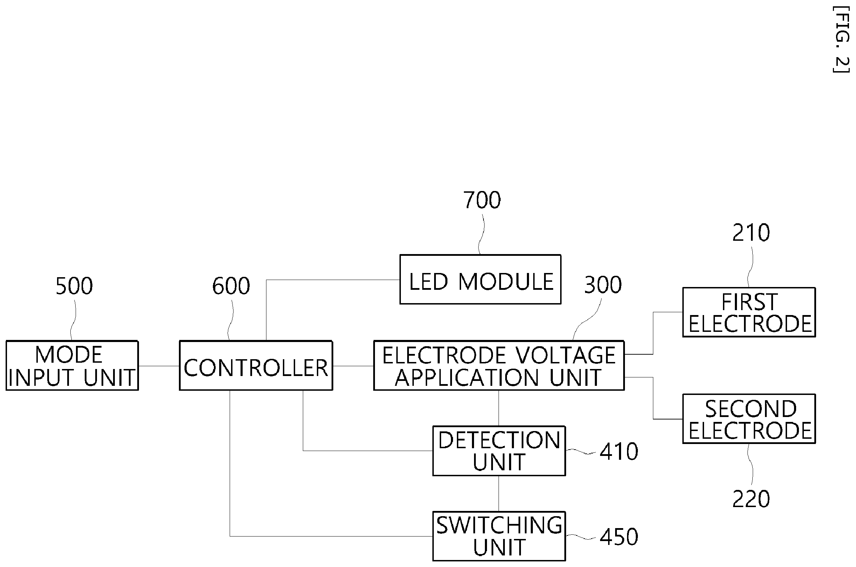

FIG. 1 is a view illustrating a skincare device according to an embodiment of the present invention, and FIG. 2 is a view for explaining a configuration of a skincare device according to an embodiment of the present invention.

Referring to FIGS. 1 and 2, the skincare device 10 according to the present embodiment may include a body 100, a first electrode 210, a second electrode 220, an electrode voltage application unit 300, a detection unit 410, a switching unit 450, a mode input unit 500, a controller 600, and an LED module 700.

The body 100 is configured to support the first electrode 210, the second electrode 220, and the like installed thereon.

The body 100 may include a base part 110 gripped by a user and a head part 130 contacting the skin. The head part 130 is formed at one end of the base part 110.

The base part 110 may extend in one direction, and the head part 130 may extend in a direction inclined at a predetermined angle with respect to the extension direction of the base part 110.

A display such as an LCD may be provided on the base part 110 so as to be exposed. The user may visually identify information provided through the display.

A power button 112 may be provided on the base part 110 so as to be exposed. The user may turn on or off the skincare device 10 through the power button 112.

A speaker 113, a charging terminal 114, and the like may be installed on the base part 110.

A rechargeable battery (not shown) may be installed in the base part 110. An inverter (not shown) for converting direct current into alternating current may be installed in the base part 110.

The first electrode 210 and the second electrode 220 may be installed on the head part 130 in a form exposed to the outside from one surface of the head part 130. The LED module 700 may be installed on one surface of the head part 130 in a form exposed to the outside. A color lighting module 131 may be installed on the head part 130 in a form exposed to the outside. The color lighting module 131 provides an aesthetic effect.

In the present embodiment, the first electrode 210 and the second electrode 220 may each have a ring shape. In this case, the first electrode 210 may be disposed in an edge region of one surface of the head part 130, and the second electrode 220 may be disposed in a central region of a front surface of the head part 130. The first electrode 210 and the second electrode 220 are insulated from each other.

The LED module 700 may be disposed at a center of the ring-shaped second electrode 220. When the color lighting module 131 has a ring shape, the color lighting module 131 may be disposed between the first electrode 210 and the second electrode 220. In this case, space utilization on one surface of the head part 130 may be maximized.

The electrode voltage application unit 300 selectively implements one of a first mode in which a first voltage is applied between the first electrode 210 and the second electrode 220 and a second mode in which a second voltage is applied to at least one of the first electrode 210 and the second electrode 220.

The first mode may be referred to as a bipolar mode in that the first voltage is applied between the first electrode 210 and the second electrode 220, and the second mode may be referred to as a unipolar mode in that the second voltage is applied to the first electrode 210 or the second electrode 220.

The first voltage is lower than the second voltage.

The first voltage is relatively low compared to the second voltage. The first voltage may be a one-digit or two-digit voltage. For example, the first voltage may be 5 V to 50 V.

The second voltage is relatively high compared to the first voltage. The second voltage may be a three-digit or four-digit voltage. For example, the second voltage may be 500 V to 3000 V.

In the first mode, the first voltage, which is a low voltage, is applied between the first electrode 210 and the second electrode 220. In the first mode, when both the first electrode 210 and the second electrode 220 contact the skin, the skin serves as a current path, and current flows through the first electrode 210, the skin, and the second electrode 220.

In the first mode, the current generated through skin contact properly stimulates a region relatively close to the epidermis of the skin, thereby improving skin health.

In the second mode, the second voltage is applied to at least one of the first electrode 210 and the second electrode 220. In the second mode, when the electrode, to which the second voltage is applied, among the first electrode 210 and the second electrode 220 contacts the skin, the electrode to which the second voltage is applied provides electrical stimulation toward the skin.

In the second mode, the electrical stimulation through skin contact is transmitted to a relatively deep region of the skin. The electrical stimulation by the high voltage causes microporation in the skin, thereby helping active ingredients of a skincare product penetrate deeply into the skin.

The electrode voltage application unit 300 described above

The detection unit 410 detects whether the first electrode 210 and the second electrode 220 are in contact with the skin of the user in the first mode.

The switching unit 450 switches the detection unit 410 in the second mode so that the detection unit 410 detects whether the skin is in contact with the electrode, to which the second voltage is applied, among the first electrode 210 and the second electrode 220.

As described above, the detection unit 410 used to detect whether the skin is in contact in the first mode may also be used to detect whether the skin is in contact in the second mode, so that a structure of the skincare device 10 is simplified and manufacture thereof is facilitated.

The mode input unit 500 is configured to receive, from the user, a selected mode selected from the first mode and the second mode. For example, the mode input unit 500 may be a mode input button 510 provided on the base part 110 so as to be exposed. The user inputs the selected mode selected from the first mode and the second mode through the mode input button 510. However, various forms of the mode input unit 500 may of course be used.

The controller 600 controls the electrode voltage application unit 300 and the switching unit 450 according to the selected mode input by the user.

For example, when the selected mode input by the user is the first mode, the controller 600 controls the electrode voltage application unit 300 so that the electrode voltage application unit 300 implements the first mode, and controls the switching unit 450 so that the detection unit 410 detects whether the skin is in contact in the first mode.

Alternatively, when the selected mode input by the user is the second mode, the controller 600 controls the electrode voltage application unit 300 so that the electrode voltage application unit 300 implements the second mode, and controls the switching unit 450 so that the detection unit 410 detects whether the skin is in contact in the second mode.

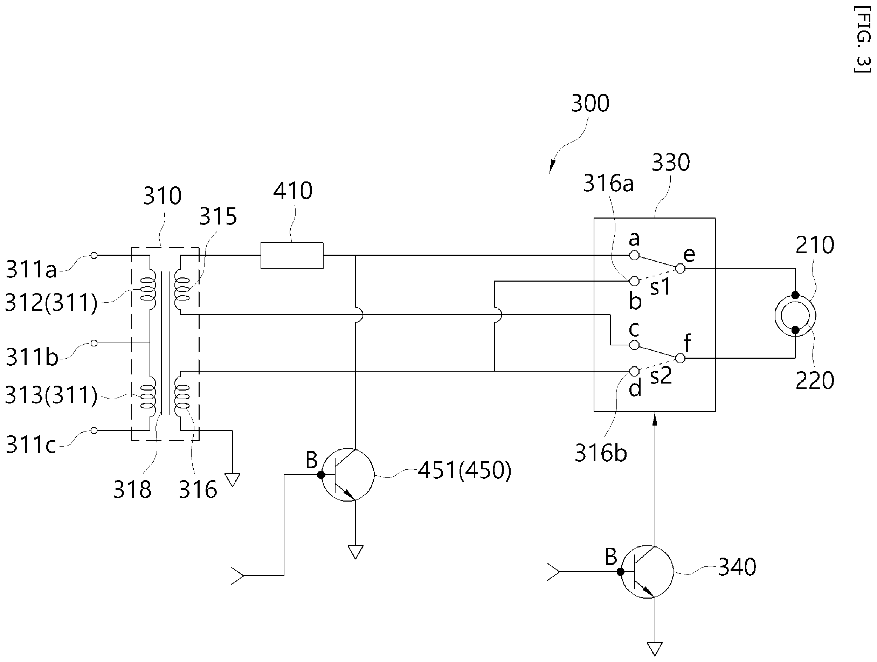

FIG. 3 is a circuit diagram of an electrode voltage application unit, a detection unit, and a switching unit according to a first embodiment of the present invention.

Referring to FIGS. 2 and 3, the electrode voltage application unit 300 may include a transformer 310, a relay 330, and a first transistor 340.

The transformer 310 includes a first coil 311 to which an input voltage is applied, a second coil 315 configured to output a first voltage induced by the first coil 311, and a third coil 316 configured to output a second voltage induced by the first coil 311, wherein one output terminal of the third coil 316 is grounded. The input voltage is an alternating current voltage.

The first voltage is determined by a turns ratio between the first coil 311 and the second coil 315, and the second voltage is determined by a turns ratio between the first coil 311 and the third coil 316.

The first coil 311, the second coil 315, and the third coil 316 share a core 318. In this case, the first voltage and the second voltage may be selectively output by a single transformer 310, so that a structure of the skincare device 10 is simplified and miniaturization is enabled as compared with a case in which separate transformers are used to output the first voltage and the second voltage.

In the present embodiment, the first coil 311 may include a first-1 coil 312 and a first-2 coil 313. The first-1 coil 312 and the first-2 coil 313 are connected in series. Input terminals of the first coil 311 may be three corresponding to the first-1 coil 312 and the first-2 coil 313.

For convenience of description, the three input terminals shown in FIG. 3 are referred to as a first input terminal 311a, a second input terminal 311b, and a third input terminal 311c. The first input terminal 311a and the second input terminal 311b serve as input terminals of the first-1 coil 312, and the second input terminal 311b and the third input terminal 311c serve as input terminals of the first-2 coil 313. The first input terminal 311a and the third input terminal 311c may serve as input terminals of the entire first coil 311.

In the present embodiment, the input voltage may be applied between any two selected from the first to third input terminals. For example, the input voltage may be applied between the first input terminal 311a and the second input terminal 311b, between the second input terminal 311b and the third input terminal 311c, or between the first input terminal 311a and the third input terminal 311c.

When the input voltage is applied between the first input terminal 311a and the second input terminal 311b, the first voltage induced by the first-1 coil 312 may be output from the second coil 315, and the second voltage induced by the first-1 coil 312 may be output from the third coil 316.

When the input voltage is applied between the second input terminal 311b and the third input terminal 311c, the first voltage induced by the first-2 coil 313 may be output from the second coil 315, and the second voltage induced by the first-2 coil 313 may be output from the third coil 316.

The relay 330 serves as a switch for selectively applying the first voltage and the second voltage.

The relay 330 includes a first movable contact (a), a second movable contact (c), a third movable contact (b), a fourth movable contact (d), a first fixed contact (e), and a second fixed contact (f).

The first movable contact (a) and the second movable contact (c) are respectively connected to two output terminals of the second coil 315.

The second movable contact (c) and the fourth movable contact (d) are respectively connected to a first branch output terminal 316a and a second branch output terminal 316b branched from another output terminal of the third coil 316. The first fixed contact (e) and the second fixed contact (f) are respectively connected to the first electrode 210 and the second electrode 220.

For reference, the relay 330 shown in FIG. 3 illustrates an initial state in which no current flows through a relay coil of the relay 330, and it is assumed that, in the initial state, the first movable contact (a) and the second movable contact (c) are respectively connected to the first fixed contact (e) and the second fixed contact (f).

The first transistor 340 grounds the relay coil (not shown) of the relay 330 connected to a direct current power source. An emitter of the first transistor 340 may be connected to the relay coil, and a collector of the first transistor 340 may be grounded.

A control signal is received at a base B of the first transistor 340.

For example, when an off signal is received at the base B of the first transistor 340, no current flows through the relay coil (not shown) of the relay 330, and thus the first movable contact (a) is connected to the first fixed contact (e) and the second movable contact (c) is connected to the second fixed contact (f) by a first switch piece S1 and a second switch piece S2 indicated by solid lines in FIG. 3, so that the first mode is implemented.

Here, receiving the off signal at the base B of the first transistor 340 includes a case in which an on signal is not received at the base B of the first transistor 340.

For example, when an on signal is received at the base B of the first transistor 340, current flows through the relay coil (not shown) of the relay 330, and thus the third movable contact (b) is connected to the first fixed contact (e) and the fourth movable contact (d) is connected to the second fixed contact (f) by the first switch piece S1 and the second switch piece S2 indicated by dotted lines in FIG. 3, so that the second mode is implemented.

According to the present embodiment, in the second mode, another output terminal of the third coil 316 is branched into the first branch output terminal 316a and the second branch output terminal 316b and is connected to the first electrode 210 and the second electrode 220. In this case, the second voltage may be applied to each of the first electrode 210 and the second electrode 220.

At this time, even when only one of the first electrode 210 and the second electrode 220 is in contact with the skin, a unipolar-type skincare effect may be obtained, which is convenient.

The controller 600 provides the control signal to the base B of the first transistor 340. The controller 600 provides the control signal to the first transistor 340 according to a selected mode input through the input unit.

For example, when the input selected mode is the first mode, the controller 600 provides the off signal to the base B of the first transistor 340. When the input selected mode is the second mode, the controller 600 provides the on signal to the base B of the first transistor 340.

The detection unit 410 is connected to one of two output terminals of the second coil 315.

The detection unit 410 detects whether the first electrode 210 and the second electrode 220 are in contact with the skin in the first mode. In the first mode, when both the first electrode 210 and the second electrode 220 come into contact with the skin, a closed loop including the first electrode 210, the skin, the second electrode 220, and the second coil 315 may be formed, and current may circulate along the closed loop.

In this case, the detection unit 410 may detect whether the first electrode 210 and the second electrode 220 are in contact with the skin by detecting the current generated due to skin contact in the first mode.

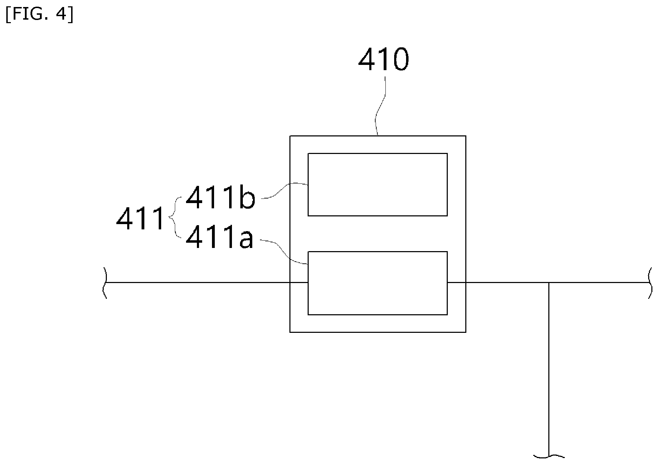

For example, the detection unit 410 may include a photocoupler 411 as shown in FIG. 4. For reference, FIG. 4 is a view illustrating an example of the detection unit shown in FIG. 3. A light emitting unit 411a of the photocoupler 411 may be connected in series with one of two output terminals of the second coil 315 (315 in FIG. 3). In the first mode, the current generated due to skin contact causes the light emitting unit 411a to emit light, and a light receiving unit 411b of the photocoupler 411 receives the emitted light from the light emitting unit 411a, thereby detecting whether the skin is in contact.

Alternatively, various known sensors for detecting current flowing along the second coil 315, the first electrode 210, and the second electrode 220 may be used by being connected in series with one of two output terminals of the second coil 315.

The switching unit 450 may include a second transistor 451 that grounds the detection unit 410.

For example, a collector of the second transistor 451 may be connected to the detection unit 410, and an emitter of the second transistor 451 may be grounded.

A control signal is received at a base B of the second transistor 451.

For example, when an off signal is received at the base B of the second transistor 451, the second transistor 451 is in a state in which current cannot flow, and when an on signal is received at the base B of the second transistor 451, the second transistor 451 is in a state in which current can flow.

Here, receiving the off signal at the base B of the second transistor 451 includes a case in which an on signal is not received at the base B of the second transistor 451.

When the off signal is received at the base B of the second transistor 451, the detection unit 410 may be used to detect whether the skin is in contact in the first mode, and when the on signal is received at the base B of the second transistor 451, the detection unit 410 may be used to detect whether the skin is in contact in the second mode.

The controller 600 provides the control signal to the second transistor 451. The controller 600 provides the control signal to the second transistor 451 according to a selected mode input through the input unit.

For example, when the input selected mode is the first mode, the controller 600 provides the off signal to the base B of the second transistor 451. When the input selected mode is the second mode, the controller 600 provides the on signal to the base B of the second transistor 451.

In the second mode, the second voltage, which is a high voltage, is applied to the first electrode 210 and the second electrode 220. In the second mode, when at least one of the first electrode 210 and the second electrode 220 to which the second voltage is applied contacts the skin, current flowing along the third coil 316 increases, and a magnetic flux change occurs in the third coil 316.

Due to the magnetic flux change generated in the third coil 316, an induced current is generated in the second coil 315 sharing the core 318 with the third coil 316. The induced current generated in the second coil 315 may flow along the second transistor 451.

In this case, the detection unit 410 may detect whether the electrode to which the second voltage is applied is in contact with the skin in the second mode by detecting the current flowing from the second coil 315 through the second transistor 451.

According to the present embodiment, in the second mode, another output terminal of the third coil 316 is branched into the first branch output terminal 316a and the second branch output terminal 316b and is connected to the first electrode 210 and the second electrode 220. In this case, the second voltage may be applied to each of the first electrode 210 and the second electrode 220.

At this time, even when only one of the first electrode 210 and the second electrode 220 is in contact with the skin, the detection unit 410 may detect current flowing along the first transistor 340, which is effective.

Referring to FIGS. 1 and 2, the LED module 700 irradiates light onto the skin. Light irradiated from the LED module 700 to the skin improves skin health.

The LED module 700 may be formed on one surface of the head part 130 of the body 100. For example, the LED module 700 may be disposed in a central region of the second electrode 220 having a ring shape, but is not limited thereto.

In the present embodiment, the controller 600 operates the LED module 700 only when contact with the skin is detected in the first mode or the second mode, and does not operate the LED module 700 otherwise.

The LED module 700 operates to irradiate light only when both the first electrode 210 and the second electrode 220 are in contact with the skin in the first mode or when an electrode, to which the second voltage is applied, among the first electrode 210 and the second electrode 220 is in contact with the skin in the second mode.

Accordingly, it is possible to prevent light of the LED module from being irradiated into eyes of a user or surrounding persons in a state in which at least one of the first electrode and the second electrode is not in contact with the skin, thereby enabling safe use.

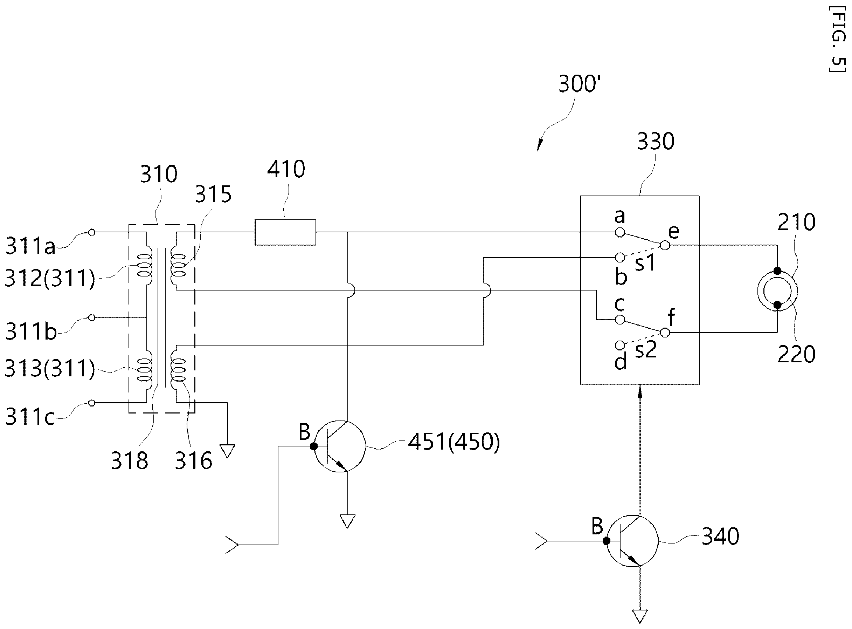

FIG. 5 is a circuit diagram illustrating an electrode voltage application unit according to a second embodiment of the present invention.

Referring to FIG. 5, the electrode voltage application unit 300 according to the second embodiment of the present invention differs from the electrode voltage application unit according to the first embodiment in that another output terminal of the third coil 316 that is not grounded is not branched and is connected to the relay 330.

Hereinafter, in describing the electrode voltage application unit 300' according to the second embodiment, description will be focused on differences from the first embodiment, and redundant description will be omitted.

The electrode voltage application unit 300' according to the second embodiment of the present invention may include a transformer 310, a relay 330, and a first transistor 340.

One output terminal of the third coil 316 included in the transformer 310 is grounded, and another output terminal thereof is connected to the relay 330. In this case, another output terminal of the third coil 316 is connected to the third movable contact (b) of the relay 330. Although not shown, another output terminal of the third coil 316 may be connected to the fourth movable contact (d) of the relay 330.

For example, when an off signal is received at the base B of the first transistor 340, no current flows through a relay coil (not shown) of the relay 330, and thus the first movable contact (a) is connected to the first fixed contact (e) and the second movable contact (c) is connected to the second fixed contact (f) by a first switch piece S1 and a second switch piece S2 indicated by solid lines in FIG. 5, so that the first mode is implemented.

And, when an on signal is received at the base B of the first transistor 340, current flows through the relay coil (not shown) of the relay 330, and thus the third movable contact (b) is connected to the first fixed contact (e) by the first switch piece (S1) indicated by dotted lines in FIG. 5, so that the second mode is implemented.

In the present embodiment, unlike the first embodiment, the second voltage is applied only to the first electrode 210 connected to another output terminal of the third coil 316 in the second mode. Although not shown, when another output terminal of the third coil 316 is connected to the fourth movable contact (d) of the relay 330, the second voltage may be applied only to the second electrode 220 in the second mode.

Although embodiments of the present invention have been described above, the scope of the present invention is not limited to the embodiments described herein, and those skilled in the art will appreciate that various modifications, additions, deletions, and substitutions are possible within the scope of the present invention, and such modifications should also be construed as falling within the scope of the present invention.

## Claims

1. A skincare device, comprising:
a first electrode and a second electrode configured to contact skin; and
an electrode voltage application unit configured to selectively implement one of a first mode in which a first voltage is applied between the first electrode and the second electrode and a second mode in which a second voltage greater than the first voltage is applied to at least one of the first electrode and the second electrode.

2. The skincare device of claim 1,
wherein the electrode voltage application unit comprises:
a transformer including a primary coil to which an input voltage is applied, a secondary coil configured to output the first voltage induced by the primary coil, and a tertiary coil configured to output the second voltage induced by the primary coil, one output terminal of the tertiary coil being grounded;
a relay including a first movable contact and a second movable contact respectively connected to two output terminals of the secondary coil, a third movable contact and a fourth movable contact respectively connected to a first branch output terminal and a second branch output terminal branched from another output terminal of the tertiary coil, and a first fixed contact and a second fixed contact respectively connected to the first electrode and the second electrode; and
a first transistor having an emitter connected to a relay coil of the relay and a collector grounded,
wherein, according to a control signal received at a base of the first transistor, the first movable contact is connected to the first fixed contact and the second movable contact is connected to the second fixed contact to implement the first mode, or the third movable contact is connected to the first fixed contact and the fourth movable contact is connected to the second fixed contact to implement the second mode.

3. The skincare device of claim 1,
wherein the electrode voltage application unit comprises:
a transformer including a primary coil to which an input voltage is applied, a secondary coil configured to output the first voltage induced by the primary coil, and a tertiary coil configured to output the second voltage induced by the primary coil, one output terminal of the tertiary coil being grounded;
a relay including a first movable contact and a second movable contact respectively connected to two output terminals of the secondary coil, a third movable contact connected to another output terminal of the tertiary coil, and a first fixed contact and a second fixed contact respectively connected to the first electrode and the second electrode; and
a first transistor having an emitter connected to a relay coil of the relay and a collector grounded,
wherein, according to a control signal received at a base of the first transistor, the first movable contact is connected to the first fixed contact and the second movable contact is connected to the second fixed contact to implement the first mode, or the third movable contact is connected to one of the first fixed contact and the second fixed contact to implement the second mode.

4. The skincare device of claim 2 or 3,
wherein the first coil includes a first-1 coil and a first-2 coil connected in series,
wherein the first coil includes first to third input terminals,
wherein the first input terminal and the second input terminal serve as input terminals of the first-1 coil, and the second input terminal and the second input terminal serve as input terminals of the first-2 coil, and
wherein the input voltage is applied between any two selected from the first to third input terminals.

5. The skincare device of claim 1, further comprising:
a mode input unit configured to receive, from a user, a selected mode selected from the first mode and the second mode; and
a controller configured to control the electrode voltage application unit according to the selected mode.

6. The skincare device of claim 1, further comprising:
a detection unit configured to detect whether the first electrode and the second electrode are in contact with skin in the first mode; and
a switching unit configured to switch the detection unit in the second mode such that the detection unit detects whether an electrode, to which the second voltage is applied, among the first electrode and the second electrode is in contact with the skin.

7. The skincare device of claim 6,
wherein the detection unit includes a photocoupler, and
wherein a light emitting unit of the photocoupler is connected in series with one of two output terminals of the secondary coil.

8. The skincare device of claim 6, wherein the detection unit is connected to one of two output terminals of the secondary coil.

9. The skincare device of claim 6, wherein the switching unit includes a second transistor having a collector connected to the detection unit and an emitter grounded.

10. The skincare device of claim 9, further comprising:
a mode input unit configured to receive, from a user, a selected mode selected from the first mode and the second mode; and
a controller configured to control the switching unit according to the selected mode.
